# EUROPÄISCHE PATENTSCHRIFT

(11) **EP 3 711 687 B1**
(45) Veröffentlichungstag und Bekanntmachung des Hinweises auf die Patenterteilung: **11.01.2023**
(21) Anmeldenummer: 19163831.1
(22) Anmeldetag: 19.03.2019
(51) Int. Cl.: A61B 17/17, A61B 17/70, A61F 2/46, A61F 2/44, A61F 2/30, A61B 17/90, A61B 17/92, A61B 17/00

(54) **SET ZUR ENDOSKOPISCHEN FIXATION EINES IMPLANTATES IN EINER BANDSCHEIBE MITTELS EINES NAGELS ODER PINS**
SET FOR ENDOSCOPIC FIXING OF AN IMPLANT IN AN INTERVERTEBRAL DISC WITH A NAIL OR PIN
ENSEMBLE DE FIXATION ENDOSCOPIQUE D'UN IMPLANT DANS UN DISQUE INTERVERTÉBRAL AU MOYEN D'UN CLOU OU D'UNE BROCHE

(43) Veröffentlichungstag der Anmeldung: 23.09.2020
(73) Patentinhaber: BioTissue SA, 1201 Genève (CH)
(72) Erfinder: Endres, Michaela, 1201 Genève (CH); Krüger, Jan Philipp, 1201 Genève (CH); Schröder, Sebastian, 1201 Genève (CH)
(74) Vertreter: Sommer, Andrea

(56) Entgegenhaltungen:
- DE-A1-102010 052 113
- US-A1- 2005 216 087
- US-A1- 2008 033 432
- US-A1- 2010 016 889

## Beschreibung

### Gebiet der Erfindung

Die Erfindung bezieht sich auf ein Set zur endoskopischen Fixation eines Implantates in einer Bandscheibe mittels eines Nagels oder Pins.

### Stand der Technik

In der Wirbelsäule sitzen die Bandscheiben als Stoßdämpfer zwischen den einzelnen Wirbeln und übernehmen eine Pufferfunktion, indem sie Erschütterungen abfangen. Sie bestehen aus einem weichen Gallertkern und einem äußeren festen Faserring, der für die nötige Stabilität sorgt. Bei dauerhafter falscher oder zu starker Belastung und altersbedingter Degeneration des Gewebes kommt es oft zu einer Bandscheibenvorwölbung. Dabei drückt der Gallertkern (Nucleus pulposus) den äußeren Faserring gegen das hintere Längsband, welches die Bandscheibe vom Rückenmarkskanal abschirmt. Nach und nach entstehen kleine Einrisse im äußeren Faserring (Annulus fibrosus). Dadurch kann ein Teil des zähflüssigen Bandscheibenkerns aus dem Ring austreten und in den Wirbelkanal gelangen. Wenn diese ausgetretene Gallertmasse dann auf eine Nervenwurzel drückt, kommt es zu massiven Schmerzen, zu Sensibilitätsstörungen oder Lähmungen. Eine chirurgische Entfernung der ausgetretenen Gallertmasse (Sequestrektomie) führt zur Dekompression der Nervenwurzeln und zur Linderung der Symptome. In 92 % der Fälle zeigt die Behandlung gute Ergebnisse. Eine Verschlechterung der Symptome ist jedoch nach 2 Jahren post-operativ zu erkennen. Der Verlust des Gewebes führt zu einem Fortschreiten der Degeneration und damit einhergehend zu einer Verringerung der Bandscheibenhöhe. Somit handelt es sich bei der Sequestrektomie um die Behandlung der Symptome (Schmerz durch Sequester), nicht aber um eine Behandlung der Ursache (Bandscheibendegeneration).

Bei einer sehr hohen Anzahl von Patienten mit der Diagnose Bandscheibenvorfall gewinnt die Entwicklung neuartiger regenerativer Therapien zur Behandlung von mesenchymalen Gewebsdefekten zunehmend an Bedeutung. Speziell im Bereich der Therapie von Bandscheibenvorfällen ist ein wachsender Bedarf an regenerativen Therapien zu verzeichnen, die die Selbstheilungskräfte stimulieren und unterstützen. Der überwiegende Anteil der Therapieoptionen sowohl innovativ als auch langjährig bewährt zielt auf den Erhalt der Bandscheibenhöhe nach Bandscheibenvorfall ab. Dabei werden Implantate zum Erhalt der Bandscheibenhöhe bzw. zum Aufbau von Reparaturgewebe in die Bandscheibe eingebracht. Ein Hauptproblem stellt hierbei das Ausdrücken (Sequestrieren) des Implantats bei Belastung dar, da der Faserring beschädigt ist und nicht heilt. Bei existierenden Operationstechniken sollen Implantate durch ein Verkleben des Faserrings oder auch durch das Verwenden eines Verschlusssystems daran gehindert werden, aus der Bandscheibe herausgedrückt zu werden. Dazu wurden Systeme wie das CE zertifizierte Barricaid^{©}oder auch das sogenannte Inclose System (Anulex Technologies, Inc., Minnetonka, MN (nicht für die EU zertifiziert)) verwendet. Das Problem des Herausdrückens des Implantates bei Belastung konnte dennoch bisher nicht zufriedenstellend gelöst werden.

Die DE 10 2010 052113 A1 offenbart verschiedene Ausführungsformen um perkutan die Fusion von Wirbelkörpern zu erzielen. Im Wesentlichen erfolgt hierbei eine Versteifung der Wirbelkörper. Es handelt sich hierbei um ein interventionelles radiologisches Verfahren, wobei gebogene Nägel, Hohlnägel oder eine gebogene Rohrkanüle in den Wirbelkörper eingebracht werden. Die Lokalisation der Nägel und/oder Kanülen wird mittels Röntgendurchleuchtungskontrolle und/oder Röntgencomputertomographie bzw. mit bildgebenden Verfahren überwacht.

### Beschreibung der Erfindung

Es ist somit die Aufgabe der vorliegenden Erfindung, ein Operationsset bereitzustellen, mit dessen Hilfe ein Implantat in eine Bandscheibe einsetzbar ist, fixiert wird und wobei ein entsprechendes Operationsverfahren zu dauerhaft guten Ergebnissen führt. Das Operationsverfahren ist nicht Teil der vorliegenden Erfindung.

Diese Aufgabe wird gelöst durch den unabhängigen Patentanspruch 1. Vorteilhafte Ausführungsformen der Erfindung gehen aus den abhängigen Patentansprüchen hervor.

Die Erfindung bezieht sich auf ein Set zur endoskopischen Fixation eines Implantates in einer Bandscheibe mittels eines Nagels oder Pins, das auf die speziellen Bedürfnisse während einer entsprechenden Bandscheibenoperation innovativ angepasst worden ist. Im Rahmen der Erfindung wurde zunächst erkannt, dass es möglich sein sollte, ein Implantat durch Nägel oder Pins, die insbesondere resorbierbar sind, in einer Bandscheibe zu fixieren. Der Erfindung liegt also die Idee für ein neues Operationsverfahren zugrunde. Dieses neue Operationsverfahren erfordert dann allerdings ein speziell darauf zugeschnittenes Operationsset.

Eine Fixation von Implantaten mittels eines resorbierbaren Nagels oder Pins ist bereits aus anderen Gebieten der Medizin bekannt. So wird eine Pinfixierung in der Orthopädie bei der Refixation von abgesprengten Teilen des Meniskus verwendet. Zudem wird sie zur Fixation von resorbierbaren Implantaten in Gelenkknorpeldefekten im Knie eingesetzt. Die Eigenschaften von Gelenkknorpel im Knie unterscheiden sich aber wesentlich vom Knorpelgewebe der Bandscheibe, so dass hier ein direkter Transfer auf Bandscheibenoperationen nicht möglich ist. Des Weiteren sind existierende Instrumentarien zur Pinfixierung beim Kniegelenk nicht dazu geeignet, eine Bandscheibenoperation, bei der ein Implantat in einer Bandscheibe mittels eines Nagels oder Pins fixiert wird, durchzuführen. Die Erfindung bezieht sich auf ein Set zur endoskopischen Fixation eines Implantates in einer Bandscheibe mittels eines Nagels oder Pins. Das Instrumentenset kann bei einer Bandscheibenoperation zusammen mit einem dabei herkömmlich verwendeten Endoskop eingesetzt werden. Das erfindungsgemäße Set weist dabei folgendes auf:
- eine Applikatorhülse, die durch ein Endoskop in eine Bandscheibe einführbar ist und durch die hindurch ein Implantat in einen Bandscheibendefekt einsetzbar ist;
- eine Bohrdrahtführungshülse, die so bemessen ist, dass sie in die Applikatorhülse einsetzbar ist; und
- einen Stößel zur Vermittlung von Schlägen bei der Fixation eines Implantates in der Bandscheibe mittels eines Nagels oder Pins, wobei der Stößel so bemessen ist, dass er in die Applikatorhülse einsetzbar ist, und
- wobei die Applikatorhülse eine abgeschrägte Spitze, einen hohlzylindrischen Hauptteil und ein flanschartiges Endstück aufweist.

Unter einer Hülse wird dabei ein länglicher, hohlzylindrischer Körper mit einer vergleichsweise dünnen Wandung verstanden. Dieser hohlzylindrische Körper kann in weiten Teilen rotationssymmetrisch um eine zentrale Längsachse sein. Dies schließt aber nicht aus, dass bestimmte Bereiche der Hülse von dieser Rotationssymmetrie abweichen und zum Beispiel spezielle Funktionalitäten erfüllen. Dies gilt insbesondere für Bereiche am proximalen bzw. distalen Ende der jeweiligen Hülse. Die Applikatorhülse ist bevorzugt einteilig ausgebildet, sie kann aber auch mehrteilig ausgebildet sein. Die Bohrdrahtführungshülse ist ebenfalls bevorzugt einteilig ausgebildet, sie kann aber auch mehrteilig ausgebildet sein.

Die Applikatorhülse ist durch ein Endoskop in eine Bandscheibe einführbar. Bevorzugt ist die Applikatorhülse auf das Endoskop aufsteckbar und/ oder es ist zur Verbindung zwischen dem Endoskop und der Applikatorhülse keine weitere Befestigung erforderlich, insbesondere keine Schraubverbindung. Bevorzugt verfügt die Applikatorhülse auch um keinen separaten Halter oder Griff, weshalb sie sehr einfach hergestellt werden kann.

Die Abmessungen der Applikatorhülse sind bevorzugt so gewählt, dass die Applikatorhülse zumindest in weiten Teilen in das Endoskop einführbar ist. Bevorzugt ist es so, dass ein Teil der Applikatorhülse nicht in das Endoskop einführbar ist, sondern einen Anschlag ausbildet, um zu verhindern, dass die gesamte Applikatorhülse in das Endoskop ungewollt hineinrutscht. Dieser Anschlag stellt keinen Halter oder Griff dar.

Ähnliches gilt für die Bohrdrahtführungshülse, die so bemessen ist, dass sie in die Applikatorhülse ganz oder teilweise einsetzbar ist. Bevorzugt ist auch hier das Vorsehen eines entsprechenden Anschlages, um zu verhindern, dass die Bohrdrahtführungshülse gänzlich in die Applikatorhülse hineinrutscht. Um ein Einsetzen der Bohrdrahtführungshülse in die Applikatorhülse zu ermöglichen, ist der Innendurchmesser der Applikatorhülse auf den Außendurchmesser der Bohrdrahtführungshülse angepasst. Bevorzugt ist dabei ein geringes Spiel im Bereich von Zehntelmillimetern vorhanden, was das Einsetzen erleichtert, wegen der Gesamtlänge von Applikatorhülse und Bohrdrahtführungshülse jedoch die Genauigkeit der Positionierung der Bohrdrahtführungshülse in der Applikatorhülse im Bereich des proximalen Endes der beiden Hülsen praktisch nicht beeinträchtigt. Der Innendurchmesser der Bohrdrahtführungshülse ist wiederum so bemessen, dass ein Bohrdraht problemlos und dennoch präzise durch die Bohrdrahtführungshülse hindurchgeführt werden kann.

Der Stößel des erfindungsgemäßen Sets ist zur Vermittlung von Schlägen bei der Fixation eines Implantates in der Bandscheibe mittels eines Nagels oder Pins geeignet und dafür bestimmt. Bevorzugt ist der Stößel deswegen kompakt und nicht als Hohlkörper ausgebildet. Dies erhöht die Stabilität und erleichtert die Vermittlung von Schlägen bzw. Hammerschlägen bei der Fixation. Des Weiteren ist der Stößel so bemessen, dass er in die Applikatorhülse einsetzbar ist. Der Außendurchmesser des Stößels ist dabei an den Innendurchmesser der Applikatorhülse entsprechend angepasst. Bevorzugt ist dabei ein geringes Spiel im Bereich von Zehntelmillimetern zwischen der Applikatorhülse und dem Stößel vorgesehen. Dies erleichtert das Einsetzen des Stößels in die Applikatorhülse, beeinträchtigt jedoch nicht die Präzision der Positionierbarkeit des Stößels. Die Länge des Stößels wird dabei bevorzugt so gewählt, dass der Stößel geringfügig länger ist als die Applikatorhülse, da bei der Vermittlung von Schlägen im Rahmen der Fixation eines Implantates in der Bandscheibe mittels eines Nagels oder eines Pins diese Schläge frei, das heißt ohne Erschütterung der Applikatorhülse, durchgeführt werden müssen. Auch der Stößel ist bevorzugt einteilig aus einem Werkstück ausgebildet, er kann aber auch mehrteilig ausgebildet sein.

Gemäß der Erfindung weist die Applikatorhülse eine abgeschrägte Spitze, einen hohlzylindrischen Hauptteil und flanschartiges Endstück auf. Die abgeschrägte Spitze ist dabei am proximalen Ende der Applikatorhülse und das flanschartige Endstück ist dabei am distalen Ende der Applikatorhülse vorgesehen. Die Applikatorhülse lässt sich hier also funktional in drei Teile untergliedern. Die Applikatorhülse kann ein- oder mehrteilig gefertigt sein. Besteht die Applikatorhülse aus mehreren Bauteilen, dann sind diese bevorzugt zusammengeschweißt oder eingeklebt. Das flanschartige Endstück ist dabei ebenfalls hohl und schließt sich insbesondere innen nahtlos an den hohlzylindrischen Hauptteil an. Der Außendurchmesser des flanschartigen Endstückes ist jedoch größer als der Außendurchmesser des hohlzylindrischen Hauptteiles, wodurch ein Hineinrutschen der Applikatorhülse in das Endoskop verhindert werden kann. Die abgeschrägte Spitze wird bevorzugt durch ein oder mehrere Aussparungen am proximalen Ende der Applikatorhülse bzw. des hohlzylindrischen Hauptteils gebildet. Es ist also bevorzugt nicht so, dass ein Außendurchmesser der Applikatorhülse im Bereich der abgeschrägten Spitze verringert würde. Der Erhalt des Außendurchmessers sorgt dafür, dass andere Bestandteile des Instrumentensets sowie auch weitere Teile, die durch die Applikatorhülse hindurchgeführt werden müssen, auch tatsächlich problemlos darin Platz finden. Dabei bietet die abgeschrägte Spitze den Vorteil, dass endoskopisch der Blick auf den Nagel oder Pin, das Implantat und den evtl. verwendeten Bohrdraht frei ist und so sichergestellt werden kann, dass der Nagel oder Pin tatsächlich das Implantat befestigt. Gemäß einer Ausführungsform der Erfindung weist die abgeschrägte Spitze mindestens ein, bevorzugt zwei oder mehrere Spitzenenden auf, die durch Aussparungen in der Applikatorhülse gebildet werden. Zusätzlich oder alternativ ist die Spitze der Applikatorhülse bezogen auf die Längsachse der Applikatorhülse rotationsunsymmetrisch abgeschrägt. Die Spitzenenden dienen dazu, die Applikatorhülse im Bandscheibenzwischenraum während der Operation durch Kontakt am angrenzenden Wirbelkörper genau zu positionieren.

Dabei sind die Spitzenenden bevorzugt stumpf ausgebildet, so dass sie zwar Halt finden, jedoch zu keinerlei Verletzung führen. Die Spitzenenden dienen zur Stabilisierung des Applikators an der Auflagestelle. Beispielsweise können die Spitzenenden in Form von stumpfen Zähnen ausgebildet sein. Sie können eine schmale, insbesondere abgerundete Anlagefläche bzw. Anlagelinie aufweisen. Die rotationsunsymmetrische Abschrägung der Spitze der Applikatorhülse erleichtert das Zusammenspiel des Instrumentensets mit dem eingesetzten Endoskop. Die rotationsunsymmetrische Abschrägung hat zur Folge, dass die Spitzenenden zumindest nicht symmetrisch in einer Ebene liegen, durch die die Längsachse der Applikatorhülse hindurch verläuft. Im einfachsten Fall werden die beiden Aussparungen im Fall von zwei Spitzenenden der abgeschrägten Spitze jeweils durch einen Schnitt mit einer Ebene geometrisch beschrieben. Ist der hohlzylindrische Hauptteil der Applikatorhülse rotationssymmetrisch, so ergeben sich bei diesem Schnitt entsprechende Ovale in der Wandung des hohlzylindrischen Hauptteils, wodurch dann die abgeschrägte Spitze überhaupt erst gebildet wird.

Eine bevorzugte Ausführungsform der Erfindung ist dadurch gekennzeichnet, dass
die abgeschrägte Spitze auf die Längsachse der Applikatorhülse rotationsunsymmetrisch abgeschrägt ist und zwei Spitzenenden aufweist; und dadurch dass
die beiden Spitzenenden und mindestens eine durch sie hindurch verlaufende Parallele zur Längsachse der Applikatorhülse mindestens eine Spitzenebene definieren;
wobei bezogen auf eine Spitzenebene eine obere Abschrägung der Spitze geometrisch durch einen Schnitt mit einer zu dieser Spitzenebene in einem oberen spitzen Winkel α geneigten oberen Ebene beschrieben wird; und/ oder
wobei bezogen auf dieselbe oder eine weitere Spitzenebene eine untere Abschrägung der Spitze geometrisch durch einen Schnitt mit einer zu dieser Spitzenebene in einem unteren spitzen Winkel β geneigten unteren Ebene beschrieben wird.

Die Herstellung einer derartigen ausgebildeten Applikatorhülse kann dadurch auf einfache Weise erfolgen. Zudem wird dadurch die vorteilhafte Rotationsunsymmetrie erreicht, was wiederum entsprechende Vorteile beim Handling des erfindungsgemäßen Sets bietet.

Ob eine Spitzenebene oder ob zwei Spitzenebenen zu definieren sind, hängt im Wesentlichen von der geometrischen Form der beiden Spitzenenden ab. Sind die beiden Spitzenenden zum Beispiel stumpf und verfügen über eine bestimmte Höhe h, so lässt sich jeweils am oberen Ende und am unteren Ende der beiden Spitzenenden mit der Höhe h eine Spitzenebene definieren. Es handelt sich in diesem Fall um zwei Spitzenebenen, an denen durch einen Schnitt mit der Applikatorhülse in den Winkeln α bzw. β eine Aussparung erzeugt werden kann, so dass die Spitze entsprechend eine obere Abschrägung und eine untere Abschrägung aufweist. Sind die beiden Spitzenenden jedoch tatsächlich spitz, so gibt es nur eine einzige Spitzenebene und diese verläuft dann auch direkt durch die Spitzen der beiden Spitzenenden. In diesem Fall stoßen auch die beiden Winkel α und β direkt aneinander an.

Gemäß einer bevorzugten Ausführungsform der Erfindung gilt für den oberen spitzen Winkel 20 °≤ α ≤ 30°, bevorzugt α = 25°. Zusätzlich oder alternativ gilt für den unteren spitzen Winkel 20° ≤ β ≤ 30°, bevorzugt β= 25°.

Gemäß einer weiteren bevorzugten Ausführungsform der Erfindung weisen die beiden Spitzenenden in einer zur Längsachse der Applikatorhülse orthogonalen Richtung eine Höhe h auf, für die gilt: 0,5 mm ≤ h ≤ 1,5 mm, bevorzugt ist h= 1,0 mm. Hierbei sind die beiden Spitzenenden also stumpf ausgebildet und können insbesondere eine schmale, gerundete Kontaktfläche bzw. einen Kontaktbogen bei Kontakt mit einer Bandscheibe ausbilden.

Gemäß einer anderen bevorzugten Ausführungsform der Erfindung weist die abgeschrägte Spitze mehrere, vorzugsweise drei bis fünf, Spitzenenden auf, die durch Aussparungen in der Applikatorhülse gebildet werden.

Gemäß einer bevorzugten Ausführungsvariante der Erfindung weist die Bohrdrahtführungshülse einen hohlzylindrischen Hauptteil und ein flanschartiges Endstück auf. Durch das flanschartige Endstück wird beim Einsetzen der Bohrdrahtführungshülse in die Applikatorhülse ein Anschlag gebildet. Das flanschartige Endstück selbst ist ebenfalls hohl und bevorzugt schließt es sich innen nahtlos an den Hohlraum im hohlzylindrischen Hauptteil der Bohrdrahtführungshülse an. Die Bohrdrahtführungshülse ist bevorzugt gleich lang oder kürzer als die Applikatorhülse inklusive der Spitzenenden.

Gemäß einer weiteren bevorzugten Ausführungsform der Erfindung weist der Stößel einen Dorn an seinem proximalen Ende, einen zylindrischen Hauptteil und ein Stößel-Kopfteil an seinem distalen Ende auf. Dabei sind bevorzugt sowohl der Dorn, der zylindrische Hauptteil als auch das Stößel-Kopfteil kompakt und somit im Wesentlichen nicht hohl ausgebildet. Der Außendurchmesser des Dorns ist dabei deutlich geringer als der Außendurchmesser des zylindrischen Hauptteils, und der Außendurchmesser des zylindrischen Hauptteils ist wiederum geringer als der Außendurchmesser des Stößel-Kopfteils. Auf diese Weise wird bei der Vermittlung von Schlägen bei der Fixation eines Implantates in der Bandscheibe mittels eines Nagels oder Pins die bei dem Schlag aufgewendete Kraft bzw. der Druck optimal auf den Kopf eines Nagels oder Pins übertragbar. Der Stößel mit seinen funktionalen drei Bestandteilen Dorn, zylindrischer Hauptteil und Stößel-Kopfteil kann ein- oder mehrteilig gefertigt sein. Bevorzugt ist der Dorn und das Hauptteil einteilig gefertigt und das Stößelkopfteil wird später separat befestigt. Bevorzugt werden die einzelnen Bestandteile des Stößels miteinander verschweißt oder geklebt, sofern sie separat gefertigt wurden.

Für die Dimensionen des Stößels bzw. seiner Teile gelten dabei bevorzugt folgende Dimensionen:
- Eine Länge LSK des Stößel-Kopfteils entlang der Längsachse des Stößels ist: 13 mm ≤ LSK ≤ 17 mm, bevorzugt LSK = 15 mm; und/oder
- Für einen Außendurchmesser DSK des Stößel-Kopfteils gilt: 8 mm ≤ DSK ≤ 12 mm, bevorzugt DSK = 10 mm; und/oder
- Für die Länge LD des Dorns entlang der Längsachse des Stößels gilt: 1,9 mm ≤ LD ≤ 2,1 mm, bevorzugt LD = 2,0 mm; und/oder
- Für den Außendurchmesser DD des Dorns gilt: 0,9 mm ≤ DD ≤ 1,1 mm, bevorzugt DD = 1,0 mm.

Der Außendurchmesser DSK des Stößel-Kopfteils erlaubt eine möglichst einfache und genaue Platzierung von Schlägen bzw. Hammerschlägen. Das zylindrische Hauptteil ist in seinen Dimensionen an die Applikatorhülse bzw. an deren Innendurchmesser angepasst. Der Dorn des Stößels ist in seinen Dimensionen an die Dimensionen eines verwendbaren Nagels bzw. Pins angepasst.

Gemäß einer weiteren bevorzugten Ausführungsvariante sind die Außendurchmesser und Innendurchmesser der Instrumente des Sets wie folgt aufeinander abgestimmt:
- Für einen Außendurchmesser DA der Applikatorhülse gilt: 3,9 mm ≤ DA ≤ 4,1 mm, bevorzugt groß DA = 4,0 mm; und
- Für einen Innendurchmesser dA der Applikatorhülse gilt: 3,3 mm ≤ dA ≤ 3,5 mm, bevorzugt dA = 3,4 mm; und
- Für einen Außendurchmesser DB der Bohrdrahtführungshülse gilt: 2,9 mm ≤ DB ≤ 3,1 mm, bevorzugt DB = 3,0 mm; und
- Für einen Innendurchmesser dB der Bohrdrahtführungshülse gilt: 2,3 mm ≤ dB ≤ 2,5 mm, bevorzugt dB = 2,4 mm; und
- Für einen Außendurchmesser DS des Stößels gilt: 2,9 mm ≤ DS ≤ 3,1 mm, bevorzugt DS = 3,0 mm.

Für eine erfolgreiche endoskopische Fixation eines Implantates in einer Bandscheibe mittels eines Nagels oder Pins sind bevorzugt auch die Längen der Applikatorhülse, der Bohrdrahtführungshülse und des Stößels aufeinander abgestimmt. Bevorzugt gelten dabei die folgenden Relationen:
- Für eine Länge LA der Applikatorhülse gilt: 250 mm ≤ LA ≤ 290 mm, bevorzugt LA = 270 mm; und
- Für eine Länge LB der Bohrdrahtführungshülse gilt: 245 mm ≤ LB ≤ 285 mm, bevorzugt LB = 265 mm; und
- Für eine Länge LS des Stößels gilt: 265 mm ≤ LS ≤ 305 mm, bevorzugt LS = 285 mm; und
- Ferner gilt gemäß dieser Ausführungsform: LB < LA < LS; wobei die angegebenen Längen LA, LB und LS bei Vorhandenseins eines flanschartigen Endstückes oder eines Kopfteils am jeweiligen Bauteil ohne das jeweilige flanschartige Endstück bzw. ohne das Kopfteil bestimmt werden. Die leichte Verkürzung der Bohrdrahtführungshülse gegenüber der Applikatorhülse sorgt dafür, dass ein durch die Bohrdrahtführungshülse eingeführter Bohrdraht beim Vorbohren eines Loches innerhalb der Bandscheibe bzw. des Wirbelkörpers genügend Freiraum besitzt. Außerdem wird dadurch die Sicht durch das Endoskop am wenigsten behindert. Der Stößel wiederum ist länger als die Applikatorhülse, um bei einer Fixation des Nagels oder Pins innerhalb der Bandscheibe die Schläge ohne Behinderung bzw. im Wesentlichen ohne Übertragung auf die Applikatorhülse ausführen zu können. Der Stößel ist ca. 2 mm bis 50 mm, bevorzugt 5 mm bis 40 mm, am meisten bevorzugt 10 mm bis 20 mm und am allermeisten bevorzugt ca. 15 mm länger als die Applikatorhülse.

Gemäß einer weiteren bevorzugten Ausführungsform der Erfindung gelten für eine Länge LFA eines flanschartigen Endstückes der Applikatorhülse entlang der Hauptachse der Applikatorhülse und für einen Außendurchmesser DFA des flanschartigen Endstückes der Applikatorhülse folgende Relationen: 2,9 mm ≤ LFA ≤ 3,1 mm, bevorzugt LFA = 3 mm; sowie 9 mm ≤ DFA ≤ 11 mm, insbesondere DFA = 10 mm.

Zusätzlich oder alternativ gelten für eine Länge LFB eines flanschartigen Endstückes der Bohrdrahtführungshülse entlang der Hauptachse der Bohrdrahtführungshülse und für einen Außendurchmesser DFB des flanschartigen Endstückes der Bohrdrahtführungshülse folgende Relationen: LFB = LFA 01, mm, bevorzugt LFB = 3 mm; sowie DFB ≥ DFA, bevorzugt DFB = 12 mm.

Ist der Außendurchmesser DFB des flanschartigen Endstückes der Bohrdrahtführungshülse geringfügig größer als der Außendurchmesser DFA der Applikatorhülse, so lässt sich, nachdem die Bohrdrahtführungshülse mit der Applikatorhülse im Anschlag bzw. vollständig bis zum Anschlag eingelegt war, die Bohrdrahtführungshülse aus der Applikatorhülse durch die leichte Erhöhung im Bereich des flanschartigen Endstückes gegenüber dem Endstück der Applikatorhülse leichter wieder entnehmen.

Gemäß einer weiteren bevorzugten Ausführungsvariante sind die Bestandteile des Sets nach einer geeigneten Sterilisation wiederverwendbar. Bevorzugt weisen die Applikatorhülse, die Bohrdrahtführungshülse und der Stößel jeweils mindestens eines der im Folgenden aufgezählten Materialien auf: vornehmlich nichtrostender Stahl, insbesondere Chirurgenstahl, wie beispielsweise die folgenden Stahlzusammensetzungen:
WNr. 1.4016 (X6Cr17), AISI 430;
WNr. 1.4021 (X20Cr13), AISI 420;
WNr. 1.4301 (X5CrNi18-10), AISI 304, (V2A), SUS304;
WNr. 1.4404 (X2CrNiMo17-12-2), AISI 316L, (V4A, A4L);
WNr. 1.4452 (X13CrMnMoN18-14-3), P2000;
insbesondere 1.4021, 1.4104, 1.4301, 1.4303, 1.4305, 1.4306, 1.4307, 1.4310, 1.4401, 1.4404, 1.4435, 1.4456, 1.4541, 1.4571, 1.4028, 1.4031, 1.4034, 1.4035, 1.4037, 1.4197, 1.4057, 1.4104, 1.4112, 1.4122, 1.4123, 1.4125, 9.9440, 1.4108, 1.4542, 1.4568 und 1.4543.

Des Weiteren können Titan und Titanwerkstoffe zu Einsatz kommen sowie sterilisierbare Kunststoffe mit entsprechender Härte. Insbesondere durch die Vermischung verschiedenster Komponenten können moderne Hochleistungspolymere, wie zum Beispiel PEEK (Polyetheretherketon-Kunststoffe) oder Keramikspritzgusskomponenten (PIM/CIM-Verfahren) mit entsprechenden Eigenschaften an Härte und Elastizität, bei entsprechender Gestaltung der Produkte eine dem Stahl vergleichbare Stabilität erreichen und somit zum Einsatz geeignet sein. Hochleistungspolymere verhalten sich bei Behandlungen mit Chemikalien, Hitze und UV-Licht den Metallen ebenfalls ähnlich. Sofern sind Kunststoffe dieser Kategorie und Qualität ebenso geeignet. Des Weiteren können die Bestandteile des erfindungsgemäßen Sets durch 3D-Druck gefertigt werden. Insofern können auch alle im 3D-Druck bekannten und verwendeten Materialien mit entsprechenden Eigenschaften verwendet werden. Bevorzugt wird Chirurgenstahl verwendet.

Gemäß einer weiteren Ausführungsvariante der Erfindung weist das Set des Weiteren folgendes auf: Einen Bohrdraht, insbesondere einen Kirschner-Draht (K-Draht) zum Vorbohren eines Loches durch ein Implantat hindurch in den knöchernen Teil eines Wirbelkörpers. Bevorzugt besteht ein solcher Bohrdraht bzw. K-Draht aus Chirurgenstahl. Es gibt ihn in unterschiedlichen Stärken. Dabei weist das vordere Ende normalerweise eine Spitze mit oder ohne Gewinde auf. Das hintere Ende kann eine Befestigungsvorrichtung zur Befestigung einer Bohrmaschine aufweisen.

Der Bohrdraht ist bevorzugt mindestens 35 mm lang. Vorteilhafterweise werden bei Verwendung des Bohrdrahtes am hinteren Ende ca. 10 mm bis 40 mm, bevorzugt ca. 35 mm zur Befestigung in einer Bohrmaschine verwendet. Am vorderen Ende kann somit die Spitze ca. 1 mm bis 25 mm, bevorzugt ca. 20 mm aus der Applikationshülse herausragen.

Gemäß einer weiteren Ausführungsform der Erfindung weist das Set des Weiteren ein Implantat zum Einbringen in eine Bandscheibe, auf.

Im Rahmen dieser Erfindung wird unter einem Implantat, ein, in den menschlichen oder tierischen Körper einpflanzbares, natürliches oder künstliches Material verstanden, das permanent oder zumindest für einen längeren Zeitraum dort verbleiben soll. Vorzugsweise besteht das Implantat aus einem resorbierbaren Material. Vorzugsweise umfasst der Begriff "Implantat" eine biologisch oder künstliche Matrix, ein biologisches oder künstliches Gewebe oder Gewebeteile oder Kombinationen davon; beispielsweise allogenes, autologes, xenogenes oder künstliches Gewebe; eine natürliche extrazelluläre Matrix oder azelluläre Matrix oder Kombinationen davon. Das Implantat kann gegebenenfalls Zellen oder Zellbestandteile umfassen. Bevorzugt wird ein textiles Implantat verwendet. Dieses Implantat kann zum Beispiel porös, schwammartig, kompakt, ein Filz, ein Vlies oder netzartig sein. Dieses Implantat ist besonders gut dazu geeignet, um innerhalb einer Bandscheibe die Bandscheibenhöhe nach einem Bandscheibenvorfall zu erhalten. Ein weiterer Vorteil besteht darin, dass Zellen zur Regeneration des Defektes einwandern und sich ansiedeln können. Typischerweise wird das Implantat durch ein Endoskopieportal und/oder die Applikatorhülse in den Bandscheibendefekt eingesetzt, beispielsweise unter Verwendung einer Fasszange.

Dabei wird das Implantat in einem Zustand, in dem es möglichst wenig Volumen einnimmt bzw. einen sehr kleinen Durchmesser aufweist, durch das Endoskopieportal und/oder die Applikatorhülse hindurchgeführt.

Gemäß einer weiteren bevorzugten Ausführungsform der Erfindung weist das Implantat wenigstens eines der folgenden Materialien auf: Kollagen; Hyaluronan (Hyaluronsäure); Polyglykolsäure; Polymilchsäure und/ oder ihre jeweiligen Kopolymere; Polycaprolacton; eine natürliche Membran, insbesondere Periost, Perichondrium und/oder Faszien, sowie deren Modifikationen; Alginat; Agarose; Fibrin; albuminhaltiges Material; Polysaccharide und/oder Kombinationen davon.

Gemäß einer weiteren Ausführungsform der Erfindung weist das Set des Weiteren mindestens einen Nagel oder Pin zur Fixation eines Implantates in einer Bandscheibe auf. Bevorzugt besteht der Nagel oder Pin aus einem resorbierbaren Material, insbesondere besteht der resorbierbare Nagel oder Pin aus einem biologisch abbaubaren Material, wie beispielweise einem Polymer aus Polymilchsäure (PLA).

Die in dem Set vorhandenen, bevorzugt resorbierbaren, Nägel oder Pins sind geometrisch so bemessen, dass sie für die avisierte Verwendung zur Fixation eines Implantates, in einer Bandscheibe besonders geeignet sind. Bevorzugt weist ein Nagel oder Pin einen Durchmesser DN auf, für den gilt: 1,4 mm ≤ DN ≤ 1,6 mm, bevorzugt ist der Durchmesser DN = 1,5 mm. Zusätzlich oder alternativ hat ein Nagel oder Pin eine Länge LN, für die folgende Relation gilt: 10 mm ≤ LN ≤ 30 mm. Am meisten bevorzugt wird ein resorbierbarer Nagel oder Pin mit den vorgenannten Dimensionen verwendet.

Die vorgenannten Ausführungsbeispiele der Erfindung können ganz oder teilweise miteinander kombiniert werden, sofern sich dabei keine technischen Widersprüche ergeben.

Die Erfindung wird noch besser verstanden werden unter Bezugnahme auf die beigefügten Figuren:
- Fig. 1:: zeigt eine Applikatorhülse eines erfindungsgemäßen Sets;
- Fig. 2:: zeigt eine Bohrdrahtführungshülse eines erfindungsgemäßen Sets;
- Fig. 3:: zeigt einen Stößel eines erfindungsgemäßen Sets;
- Fig. 4:: zeigt eine Tabelle mit typischen Maßen einer Applikatorhülse, einer Bohrdrahtführungshülse und eines Stößels für ein erfindungsgemäßes Set;
- Fig. 5:: zeigt beispielhafte Bemaßungen inklusive Toleranzen für ein erfindungsgemäßes Set mit Applikatorhülse, Bohrdrahtführungshülse und Stößel.

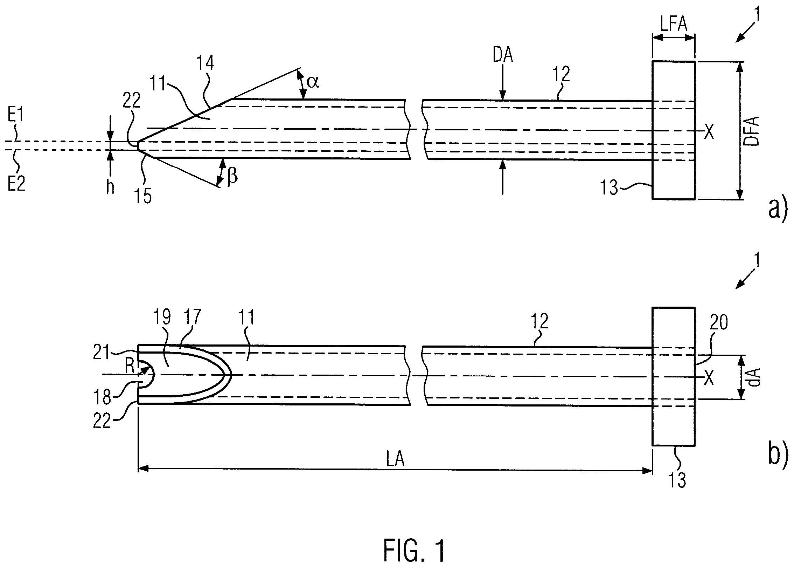

Fig. 1 zeigt beispielhaft eine Applikatorhülse 1 eines erfindungsgemäßen Sets zur endoskopischen Fixation eines Implantates in einer Bandscheibe mittels eines Nagels oder Pins. Dabei zeigt Darstellung 1a) eine seitliche Schnittansicht der Applikatorhülse 1 durch die Längsachse X. Figur 1b) zeigt eine um 90° dazu gedrehte Schnittdarstellung durch die Längsachse X in Draufsicht. Die Applikatorhülse 1 verfügt über einen hohlzylindrischen Hauptteil 12, wobei am proximalen Ende der Applikatorhülse 1 eine abgeschrägte Spitze 11 angeordnet ist. Am distalen Ende des hohlzylindrischen Hauptteils 12 befindet sich ein flanschartiges Endstück 13. Die abgeschrägte Spitze 11 wird durch Aussparungen 18 und 19 im Zylindermantel des hohlzylindrischen Hauptteils 12 generiert. Die geometrischen Verhältnisse sind in Fig. 1a) genauer dargestellt. Die abgeschrägte Spitze 11 verfügt über zwei Spitzenenden 21 und 22. In Fig. 1a) liegen diese beiden Spitzenenden deckungsgleich hintereinander, so dass in der Darstellung in Fig. 1a) nur das Spitzenende 22 zu erkennen ist. Die beiden Spitzenenden 21, 22 verfügen über jeweils einen Zahn der Höhe h, der durch den Abschluss des Zylindermantels der Applikatorhülse 1 gebildet wird. Die abgeschrägte Spitze 11 ist hinsichtlich der Längsachse X der Applikatorhülse 1 rotationsunsymmetrisch abgeschrägt. Die beiden Zähne 21, 22 liegen im gezeigten Beispiel in Fig. 1a) deshalb unterhalb der Längsachse X der Applikatorhülse 1. Die beiden Spitzenenden 21, 22 und durch sie hindurch verlaufende Parallelen zur Längsachse X der Applikatorhülse 1 definieren Spitzenebenen E1, E2. Die Ebenen E1 und E2 werden jeweils am oberen Ende der Spitzenenden 21, 22 bzw. an unteren Ende der Spitzenenden 21, 22 gedanklich definiert. Bezogen auf die Spitzenebene E1 wird eine obere Abschrägung 14 der Spitze 11 geometrisch durch einen Schnitt mit einer zu dieser Spitzenebene E1 in einem oberen spitzen Winkel α geneigten oberen Ebene 14 beschrieben. Des Weiteren wird bezogen auf die Spitzenebene E2 eine untere Abschrägung 15 der Spitze 11 geometrisch durch einen Schnitt mit einer zu der Spitzenebene E2 in einem unteren spitzen Winkel β geneigten unteren Ebene 15 beschrieben. Das Ergebnis des durchgeführten Schnittes sind die Aussparungen 18 und 19, die in Fig. 1b) am besten zu erkennen sind.

In Fig. 1 sind des Weiteren die inneren und äußeren Durchmesser der Applikatorhülse 1 eingezeichnet. Die wirksame Länge LA der Applikatorhülse 1 ergibt sich in Richtung der Längsachse gemessen vom Spitzenende bis hin zum Beginn des flanschartigen Endstücks 13. Das flanschartige Endstück verfügt über einen entsprechend größeren äußeren Durchmesser DFA sowie über eine zusätzliche Länge LFA. Der Innendurchmesser im flanschartigen Endstück 13 ist derselbe wie innerhalb des hohlzylindrischen Hauptteiles 12.

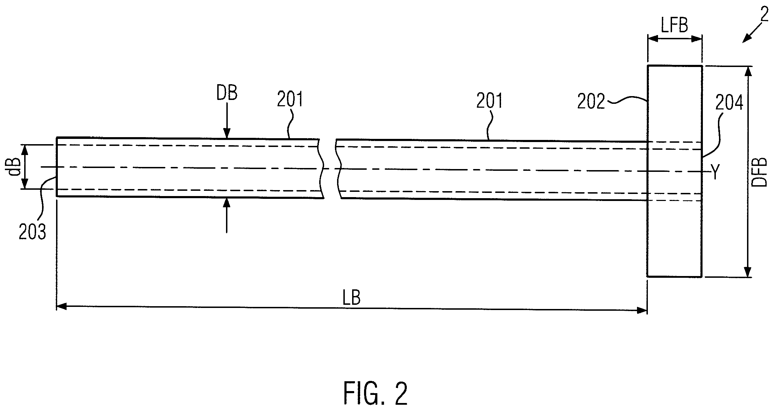

Fig. 2 zeigt eine Bohrdrahtführungshülse 2 eines erfindungsgemäßen Sets zur endoskopischen Fixation eines Implantates in einer Bandscheibe mittels eines Nagels oder Pins. Die Bohrdrahtführungshülse 2 umfasst einen hohlzylindrischen Hauptteil 201 sowie ein flanschartiges Endstück 202. Die Bohrdrahtführungshülse 2 verfügt über einen Innendurchmesser dB sowie über einen Außendurchmesser DB. Die wirksame Länge LB der Bohrdrahtführungshülse 2 ist bevorzugt etwas kürzer als die wirksame Länge LA der Applikatorhülse 1 in Fig. 1. Die Bohrdrahtführungshülse 2 aus Fig. 2 weist an ihrem proximalen Ende eine Öffnung 203 auf. An ihrem distalen Ende verfügt sie über eine Öffnung 204 in dem flanschartigen Endstück 202 der Bohrdrahtführungshülse 2. Der Außendurchmesser DFB der Bohrdrahtführungshülse 2 ist dabei im gezeigten Ausführungsbeispiel geringfügig größer als der Außendurchmesser DFA des flanschartigen Endstückes 13 der Applikatorhülse 1. Dies ermöglicht eine einfachere Separation der von Bohrdrahtführungshülse 2 und Applikatorhülse 1, nachdem die beiden miteinander im Anschlag waren.

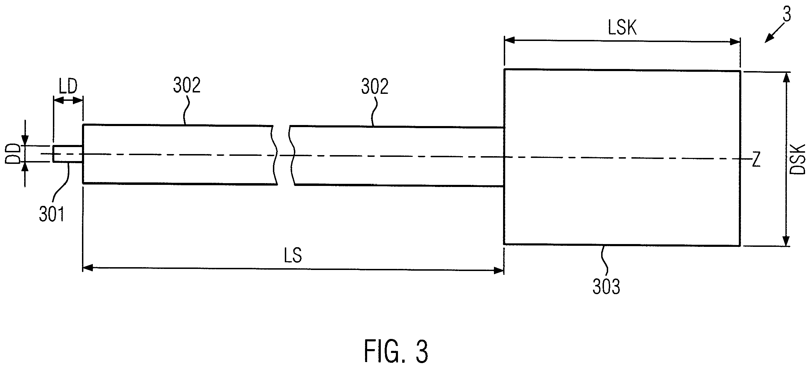

Fig. 3 zeigt einen Stößel für ein Set zur endoskopischen Fixation eines Implantates in einer Bandscheibe. Der dargestellte Stößel 3 ist einstückig ausgebildet und verfügt über drei unterschiedliche funktionale Bereiche: Einen Dorn 301 am proximalen Ende, einen zylindrischen Hauptteil 302 sowie ein Stößel-Kopfteil 303 am distalen Ende. Im gezeigten Beispiel ist der Stößel vollständig kompakt ausgebildet, was eine bessere Kraftübertragung bzw. Druckübertragung bei der Vermittlung von Schlägen bzw. Hammerschlägen zur Fixation ermöglicht. Der Außendurchmesser DS des Stößels 3 bzw. seines zylindrischen Hauptteiles 302 ist an den Innendurchmesser dA der Applikatorhülse 1 angepasst. Verglichen damit ist der Außendurchmesser DD des Dorns deutlich geringer und auch die Länge LD des Dorns entlang der Längsrichtung bzw. Achsrichtung Z des Stößels 3 ist äußerst kurz und beträgt nur wenige Millimeter. Das Stößel-Kopfteil 303 hingegen ist mit einem großen Außendurchmesser DSK versehen und weist eine Länge LSK im Bereich von einigen Millimetern auf. Das Stößel-Kopfteil 303 ist also hinreichend groß und stabil ausgebildet, um bei Schlageinwirkung hinreichend stabil zu sein und eine zur Längsachse Z rechtwinklige Fläche für das Schlaginstrument darzubieten, die groß genug als Target für Schläge ist.

Die Verwendung eines erfindungsgemäßen Sets zur endoskopischen Fixation eines Implantates in einer Bandscheibe mittels eines Nagels oder Pins während einer Operation kann beispielsweise folgendermaßen erfolgen: Zunächst wird die Applikatorhülse 1 durch einen Endoskopieport in die Bandscheibe eingeführt. Anschließend wird das Implantat durch die Applikatorhülse 1 in den Bandscheibendefekt eingesetzt, beispielsweise unter Verwendung einer Fasszange. Nach Entfernung der Fasszange wird die Bohrdrahtführungshülse 2 in die Applikatorhülse 1 eingesetzt. Mittels eines Bohrdrahtes, insbesondere eines K-Drahtes, wird ein Loch durch das Implantat in den knöchernen Teil des angrenzenden Wirbelkörpers gebohrt. Anschließend wird der Bohrdraht herausgezogen und die Bohrdrahtführungshülse 2 wird aus der Applikatorhülse 1 ebenfalls herausgezogen. Dabei sollte der Winkel zum Bohrloch nicht mehr verändert werden. In einem nächsten Schritt wird ein resorbierbarer Nagel oder Pin in die Applikatorhülse 1 eingeführt, der bis zum vorgebohrten Loch durch die Hülse 1 rutscht. Mittels des eingeführten Stößels 3 kann unter kurzen Hammerschlägen der Nagel in das vorgebohrte Loch im Wirbelkörper eingeschlagen werden. Somit fixiert der Kopf des Nagels das Implantat in der Bandscheibe.

Die Längenverhältnisse von Applikatorhülse 1, Bohrdrahtführungshülse und Stößel 3 sind aufeinander abgestimmt, gleiches gilt für ihre Innen- bzw. Außendurchmesser. Die in den Fig. 1, 2 und 3 dargestellten Teile des Sets sind in der dargestellten Weise miteinander kombinierbar.

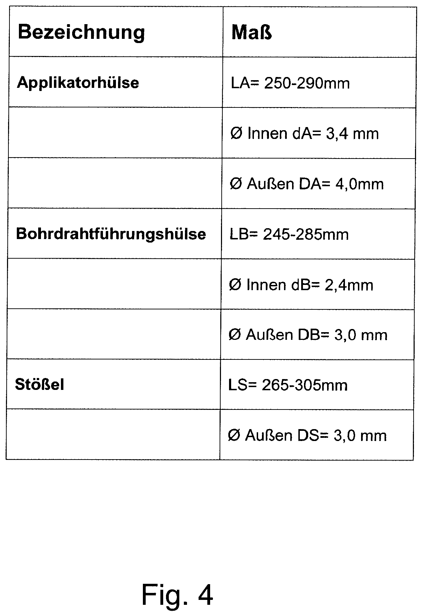

Fig. 4 fasst Längenbeziehungen und aufeinander abgestimmte Innen- bzw. Außendurchmesser von Applikatorhülse 1, Bohrdrahtführungshülse 2 und Stößel 3 noch einmal in einer entsprechenden Übersicht zusammen. Die Bohrdrahtführungshülse 2 ist dabei geringfügig kürzer als die Applikatorhülse 1 (LB geringfügig kürzer als LA). Der Stößel 3 hingegen ist entsprechend länger (LS > LA). Die Bohrdrahtführungshülse 2 und der Stößel 3 können nacheinander in die Applikatorhülse 1 eingesetzt werden. Entsprechend sind die Außendurchmesser DB der Bohrdrahtführungshülse bzw. DS des Stößels 3 im dargestellten Beispielfall mit 3,0 mm jeweils gleich bemessen. Die Wandstärken von Applikatorhülse 1 und Bohrdrahtführungshülse 2 betragen jeweils nur einige Zehntelmillimeter. Demgegenüber ist der Stößel 3 kompakt ausgebildet.

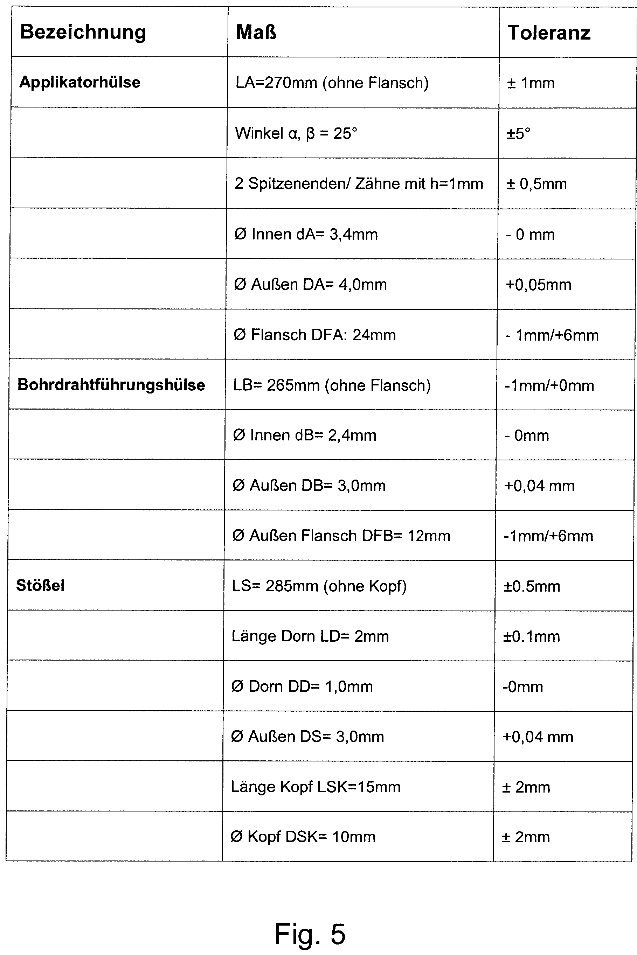

Fig. 5 zeigt in einer weiteren beispielhaften Ausführungsvariante Maße und Toleranzen von Applikatorhülse 1, Bohrdrahtführungshülse 2 und Stößel 3. Bei den angegebenen Toleranzen handelt es sich um zulässige Fertigungstoleranzen, die die Funktionsweise des erfindungsgemäßen Sets nicht beeinträchtigen. Die größte Genauigkeit wird dabei beim inneren Durchmesser dA bei der Applikatorhülse 1 und beim inneren Durchmesser dB der Bohrdrahtführungshülse 2 gefordert. Hier sollte auf den Zehntelmillimeter genau gefertigt werden. Der Toleranzbereich beim Außendurchmesser DB der Bohrdrahtführungshülse 2 ist geringfügig größer und beträgt + 0,04 mm. Entsprechendes gilt für den Außendurchmesser DS des Stößels 3. Nur dann kann gewährleistet werden, dass die Bohrdrahtführungshülse 2 bzw. der Stößel 3 ohne Verkanten oder Reibungseffekte durch die Applikatorhülse 1 hindurchgeschoben bzw. in diese eingesetzt werden kann.

Mithilfe der Erfindung, ist es erstmals gelungen, ein Set zur endoskopischen Fixation eines Implantates in einer Bandscheibe mittels eines Nagels oder Pins bereitzustellen. Dadurch werden bei Bandscheibenoperationen langfristig deutlich bessere Erhaltungseffekte der Bandscheibenhöhe erhofft.

## Patentansprüche

1. Set zur endoskopischen Fixation eines Implantates in einer Bandscheibe mittels eines Nagels oder Pins, das folgendes aufweist:
eine Applikatorhülse (1), die durch ein Endoskop in eine Bandscheibe einführbar ist und durch die hindurch ein Implantat in einen Bandscheibendefekt einsetzbar ist;
eine Bohrdrahtführungshülse (2), die so bemessen ist, dass sie in die Applikatorhülse (1) einsetzbar ist; und
einen Stößel (3) zur Vermittlung von Schlägen bei der Fixation eines Implantates in der Bandscheibe mittels eines Nagels oder Pins, wobei der Stößel (3) so bemessen ist, dass er in die Applikatorhülse (1) einsetzbar ist,und
wobei die Applikatorhülse (1) eine abgeschrägte Spitze (11), einen hohlzylindrischen Hauptteil (12) und ein flanschartiges Endstück (13) aufweist,

2. Set gemäß dem vorangehenden Anspruch,
wobei die abgeschrägte Spitze (11) mindestens zwei Spitzenenden (21, 22) aufweist, die durch Aussparungen (17, 18) in der Applikatorhülse (1) gebildet werden; und/ oder
wobei die Spitze (11) der Applikatorhülse (1) bezogen auf die Längsachse (X) der Applikatorhülse (1) rotationsunsymmetrisch abgeschrägt ist.

3. Set gemäß einem der Ansprüche 1 bis 2,
wobei die abgeschrägte Spitze (11) auf die Längsachse (X) der Applikatorhülse (1) bezogen rotationsunsymmetrisch abgeschrägt ist und zwei Spitzenenden (21,22) aufweist; und
wobei die beiden Spitzenenden (21, 22) und mindestens eine durch sie hindurch verlaufende Parallele zur Längsachse (X) der Applikatorhülse (1) mindestens eine Spitzenebene (E1, E2) definieren;
wobei bezogen auf eine Spitzenebene (E1) eine obere Abschrägung (14) der Spitze (11) geometrisch durch einen Schnitt mit einer zu dieser Spitzenebene (E1) in einem oberen spitzen Winkel α geneigten oberen Ebene (14) beschrieben wird; und/oder
wobei bezogen auf dieselbe oder eine weitere Spitzenebene (E2) eine untere Abschrägung (15) der Spitze (11) geometrisch durch einen Schnitt mit einer zu dieser Spitzenebene (E2) in einem unteren spitzen Winkel β geneigten unteren Ebene (15) beschrieben wird.

4. Set gemäß dem vorangehenden Anspruch,
wobei für den oberen spitzen Winkel α gilt: 20° ≤ α ≤ 30°, insbesondere α = 25°; und/ oder
wobei für den unteren spitzen Winkel β gilt: 20° ≤ β ≤ 30°, insbesondere β = 25°.

5. Set gemäß einem der Ansprüche 2 bis 4, wobei die beiden Spitzenenden (21, 22) in einer zur Längsachse (X) der Applikatorhülse (1) orthogonalen Richtung eine Höhe h aufweisen, für die gilt:
0,5mm ≤ h ≤ 1,5mm, insbesondere h=1,0mm.

6. Set gemäß einem der vorangehenden Ansprüche, wobei die Bohrdrahtführungshülse (2) einen hohlzylindrischen Hauptteil (201) und ein flanschartiges Endstück (202) aufweist.

7. Set gemäß einem der vorangehenden Ansprüche,
wobei der Stößel (3) einen Dorn (301) an seinem proximalen Ende, einen zylindrischen Hauptteil (302) und ein Stößel-Kopfteil (303) an seinem distalen Ende aufweist; und/ oder
wobei für eine Länge LSK des Stößel-Kopfteils (303) entlang der Längsachse (Z) des Stößels (3) gilt: 13mm ≤ LSK ≤ 17mm, insbesondere LSK=15mm; und/ oder
wobei für einen Außendurchmesser DSK des Stößel-Kopfteils (303) gilt: 8mm ≤ DSK ≤ 12mm, insbesondere DSK=10mm; und/ oder
wobei für die Länge LD des Dorns (301) entlang der Längsachse (Z) des Stößels (3) gilt:
1,9mm ≤ LD ≤ 2,1 mm, insbesondere LD=2,0mm; und/ oder
wobei für den Außendurchmesser DD des Dorns (301) gilt:
0,9mm ≤ DD ≤ 1,1mm, insbesondere DD=1,0mm.

8. Set gemäß einem der vorangehenden Ansprüche,
wobei für einen Außendurchmesser DA der Applikatorhülse (1) gilt:
3,9mm ≤ DA ≤ 4,1mm, insbesondere DA=4,0mm;und
wobei für einen Innendurchmesser dA der Applikatorhülse (1) gilt:
3,3mm ≤ dA ≤ 3,5mm, insbesondere dA=3,4mm; und
wobei für einen Außendurchmesser DB der Bohrdrahtführungshülse (2) gilt:
2,9mm ≤ DB ≤3,1mm, insbesondere DB=3,0mm; und
wobei für einen Innendurchmesser dB der Bohrdrahtführungshülse (2) gilt:
2,3mm ≤ dB ≤ 2,5mm, insbesondere dB=2,4mm; und
wobei für einen Außendurchmesser DS des Stößels (3) gilt:
2,9mm ≤ DS ≤ 3,1mm, insbesondere DS=3,0mm.

9. Set gemäß einem der vorangehenden Ansprüche,
wobei für eine Länge LA der Applikatorhülse (1) gilt:
250mm ≤ LA ≤ 290mm, insbesondere LA=270mm; und
wobei für eine Länge LB der Bohrdrahtführungshülse (2) gilt:
245mm ≤ LB ≤ 285mm, insbesondere LB=265mm; und
wobei für eine Länge LS des Stößels (3) gilt:
265mm ≤ LS ≤ 305mm, insbesondere LS=285mm; und
wobei ferner gilt: LB < LA < LS; und
wobei die angegebenen Längen LA, LB und LS bei Vorhandensein eines flanschartigen Endstückes (13, 202) oder eines Kopfteils (303) am jeweiligen Bauteil (1, 2, 3) ohne das jeweilige flanschartige Endstück (13, 202) oder ohne das Kopfteil (303) bestimmt werden.

10. Set gemäß einem der vorangehenden Ansprüche,
wobei für eine Länge LFA eines flanschartigen Endstückes (13) der Applikatorhülse (1) entlang der Hauptachse (X) der Applikatorhülse (1) und für einen Außendurchmesser DFA des flanschartigen Endstückes (13) der Applikatorhülse (1) gilt: 2,9mm ≤ LFA ≤ 3,1mm, insbesondere LFA=3mm, sowie 9mm ≤ DFA ≤ 11mm, insbesondere DFA=10mm; und/ oder
wobei für eine Länge LFB eines flanschartigen Endstückes (202) der Bohrdrahtführungshülse (2) entlang der Hauptachse (Y) der Bohrdrahtführungshülse (2) und für einen Außendurchmesser DFB des flanschartigen Endstückes (202) der Bohrdrahtführungshülse (2) gilt: LFB = LFA±0,1 mm, insbesondere LFB=3mm, sowie DFB ≥ DFA, insbesondere DFB=12mm.

11. Set gemäß einem der vorangehenden Ansprüche, wobei die Bestandteile des Sets nach einer geeigneten Sterilisation wiederverwendbar sind.

12. Set gemäß einem der vorangehenden Ansprüche, das des Weiteren Folgendes aufweist:
einen Bohrdraht, insbesondere einen K-Draht, zum Vorbohren eines Loches durch ein Implantat hindurch in den knöchernen Teil eines Wirbelkörpers.

13. Set gemäß einem der vorangehenden Ansprüche, das des Weiteren Folgendes aufweist:
ein Implantat zum Einbringen in eine Bandscheibe.

14. Set gemäß dem vorangehenden Anspruch, wobei das Implantat porös, schwammartig, kompakt, ein Filz, ein Vlies oder netzartig ist.

15. Set gemäß einem der Ansprüche 13 bis 14, wobei das Implantat wenigstens eines der folgenden Materialien aufweist:
- Kollagen;
- Hyaluronan (Hyaluronsäure);
- Polyglykolsäure, Polymilchsäure und/ oder ihre jeweiligen Kopolymere;
- Polycaprolacton;
- eine natürliche Membran, insbesondere Periost, Perichondrium und/ oder Faszien, sowie deren Modifikationen;
- Alginat;
- Agarose;
- Fibrin;
- Albuminhaltiges Material;
- Polysaccharide
sowie Kombinationen davon.

16. Set gemäß einem der vorangehenden Ansprüche, das des Weiteren Folgendes aufweist:
mindestens einen, bevorzugt resorbierbaren, Nagel oder Pin zur Fixation eines Implantates in einer Bandscheibe.

17. Set gemäß dem vorangehenden Anspruch,
wobei der, bevorzugt resorbierbare, Nagel oder Pin einen Durchmesser DN aufweist, für den gilt: 1,4mm ≤ DN ≤ 1,6mm, insbesondere DN=1,5mm; und/ oder
wobei der resorbierbare Nagel oder Pin eine Länge LN aufweist, für die gilt: 10mm ≤ LN ≤30mm.

18. Set gemäß einem der vorangehenden Ansprüche,
wobei die Applikatorhülse (1) auf ein Endoskop aufsteckbar ist; und/ oder
wobei zur Verbindung zwischen einem Endoskop und der Applikatorhülse (1) keine weitere Befestigung, insbesondere keine Schraubverbindung, erforderlich ist; und/ oder
wobei die Applikatorhülse (1) keinen separaten Halter oder Griff aufweist.

## Claims

1. A set for the endoscopic fixation of an implant in an intervertebral disk by means of a nail or pin comprising:
an applicator sheath (1) which is insertable through an endoscope into an intervertebral disk and through which an implant is insertable into an intervertebral disk defect;
a drill wire guide sheath (2) sized to be insertable into the applicator sheath (1); and
a pusher (3) for mediating blows during fixation of an implant in the intervertebral disk by means of a nail or pin, wherein the pusher (3) being sized to be insertable into the applicator sheath (1) and
wherein the applicator sheath (1) comprises a beveled tip (11), a hollow cylindrical main part (12) and a flange-like end piece (13).

2. The set according to any of the preceding claims,
wherein the beveled tip (11) comprises at least two tip ends (21, 22) formed by recesses (17, 18) in the applicator sheath (1); and/ or
wherein the tip (11) of the applicator sheath (1) is rotationally asymmetrically beveled with respect to the longitudinal axis (X) of the applicator sheath (1).

3. The set according to any of claims 1 to 2,
wherein the beveled tip (11) is rotationally asymmetrically beveled with respect to the longitudinal axis (X) of the applicator sheath (1) and has two tip ends (21, 22); and
wherein both tip ends (21, 22) and at least one line passing therethrough which is parallel with respect to the longitudinal axis (X) of the applicator sheath (1) define at least one tip plane (E1, E2);
wherein, with respect to a tip plane (E1), an upper bevel (14) of the tip (11) is geometrically described by an intersection with an upper plane (14) inclined at an upper acute angle α with respect to said tip plane (E1); and/or
wherein, with respect to the same or a further tip plane (E2), a lower bevel (15) of the tip (11) is geometrically described by an intersection with a lower plane (15) inclined at a lower acute angle β with respect to said tip plane (E2).

4. The set according to the preceding claim,
wherein for the upper acute angle α the following applies: 20° ≤ α ≤ 30°, in particular α = 25°; and/ or
wherein for the lower acute angle β the following applies: 20° ≤ β ≤ 30°, in particular β = 25°.

5. The set according to any of claims 2 to 4, wherein both tip ends (21, 22) have, in a direction orthogonal to the longitudinal axis (X) of the applicator sheath (1), a height h for which holds:
0.5 mm ≤ h ≤ 1.5 mm, in particular h = 1.0 mm.

6. The set according to any of the preceding claims, wherein the drill wire guide sheath (2) comprises a hollow cylindrical main part (201) and a flange-like end piece (202).

7. The set according to any of the preceding claims,
wherein the pusher (3) comprises a mandrel (301) at its proximal end; a cylindrical main part (302) and a pusher header (303) at its distal end; and/ or
wherein for a length LSK of the pusher header (303) along the longitudinal axis (Z) of the pusher (3) the following applies: 13 mm ≤ LSK ≤ 17 mm, in particular LSK = 15 mm; and/ or
wherein for an outer diameter DSK of the pusher header (303) the following applies: 8 mm ≤ DSK ≤ 12 mm, in particular DSK = 10 mm; and/ or
wherein for the length LD of the mandrel (301) along the longitudinal axis (Z) of the pusher (3) the following applies:
1.9 mm ≤ LD ≤ 2.1 mm, in particular LD = 2.0 mm; and/ or
wherein for the outer diameter DD of the mandrel (301) the following applies: 0.9 mm ≤ DD ≤ 1.1 mm, in particular DD = 1.0 mm.

8. The set according to any of the preceding claims,
wherein for an outer diameter DA of the applicator sheath (1) the following applies: 3.9 mm ≤ DA ≤ 4.1 mm, in particular DA = 4.0 mm; and
wherein for an inner diameter dA of the applicator sheath (1) the following applies: 3.3 mm ≤ dA ≤ 3.5 mm, in particular dA = 3.4 mm; and
wherein for an outer diameter DB of the drill wire guide sheath (2) the following applies:
2.9 mm ≤ DB ≤ 3.1 mm, in particular DB = 3.0 mm; and
wherein for an inner diameter dB of the drill wire guide sheath (2) the following applies:
2.3 mm ≤ dB ≤ 2.5 mm, in particular dB = 2.4 mm; and
wherein for an outer diameter DS of the pusher (3) the following applies:
2.9 mm ≤ DS ≤ 3.1 mm, in particular DS = 3.0 mm.

9. The set according to any of the preceding claims,
wherein for a length LA of the applicator sheath (1) the following applies:
250 mm ≤ LA ≤ 290 mm, in particular LA = 270 mm; and
wherein for a length LB of the drill wire guide sheath (2) the following applies:
245 mm ≤ LB ≤ 285 mm, in particular LB = 265 mm; and
wherein for a length LS of the pusher (3) the following applies:
265 mm ≤ LS ≤ 305 mm, in particular LS = 285 mm; and
wherein further the following applies: LB < LA < LS; and
wherein the specified lengths LA, LB and LS are determined in the case of the presence of a flange-like end piece (13, 202) or a header (303) at the respective component (1, 2, 3) without the respective flange-like end piece (13, 202) or without the header (303).

10. The set according to any of the preceding claims,
wherein for a length LFA of a flange-like end piece (13) of the applicator sheath (1) along the main axis (X) of the applicator sheath (1) and for an outer diameter DFA of the flange-like end piece (13) of the applicator sheath (1) the following applies: 2.9 mm ≤ LFA ≤ 3.1 mm, in particular LFA = 3 mm, as well as 9 mm ≤ DFA ≤ 11 mm, in particular DFA = 10 mm; and/ or
wherein for a length LFB of a flange-like end piece (202) of the drill wire guide sheath (2) along the main axis (Y) of the drill wire guide sheath (2) and for an outer diameter DFB of the flange-like end piece (202) of the drill wire guide sheath (2) the following applies: LFB = LFA ± 0.1 mm, in particular LFB = 3 mm, as well as DFB ≥ DFA, in particular DFB = 12 mm.

11. A set according to any one of the preceding claims, wherein the components of the set are reusable after appropriate sterilization.

12. The set according to any of the preceding claims, which furthermore comprises the following:
a drill wire, in particularly a K wire, for predrilling a hole through an implant into the osseous part of a vertebral body.

13. The set according to any of the preceding claims, which furthermore comprises the following:
an implant for placing in an intervertebral disk.

14. The set according to the preceding claim, wherein the implant is porous, spongy, compact, a felt, a fleece or mesh-like.

15. The set according to any of claims 13 to 14, wherein the implant comprises at least one of the following materials:
- collagen;
- hyaluronan (hyaluronic acid);
- polyglycolic acid, polylactic acid and/ or their respective copolymers;
- polycaprolactone;
- a natural membrane, in particularly periosteum, perichondrium and/ or fasciae, as well as their modifications;
- alginate;
- agarose;
- fibrin;
- albumin containing material;
- polysaccharides
as well as combinations thereof.

16. The set according to any of the preceding claims, which furthermore comprises the following:
at least one, preferably resorbable, nail or pin for the fixation of an implant in an intervertebral disk.

17. The set according to the preceding claim,
wherein the, preferably resorbable, nail or pin has a diameter DN for which the following applies: 1.4 mm ≤ DN ≤ 1.6 mm, in particular DN = 1.5 mm; and/ or
wherein the resorbable nail or pin has a length LN for which the following applies: 10 mm ≤ LN ≤ 30 mm.

18. The set according to any of the preceding claims,
wherein the applicator sheath (1) can be plugged onto an endoscope; and/ or
wherein no further fastening, in particular no screw connection, is required for the connection between an endoscope and the applicator sheath (1); and/or
wherein the applicator sheath (1) does not comprise a separate holder or handle.

## Revendications

1. Ensemble pour la fixation endoscopique d'un implant dans un disque intervertébral au moyen d'un clou ou d'une broche, qui présente les éléments suivants :
un tube applicateur (1) qui peut être introduit dans un disque intervertébral à travers un endoscope et à travers lequel un implant peut être inséré dans un défaut du disque intervertébral ;
un tube guide-fil de forage (2) qui est dimensionné de telle sorte qu'il peut être inséré dans le tube applicateur (1) ; et
un poussoir (3) pour communiquer des chocs lors de la fixation d'un implant dans le disque intervertébral au moyen d'un clou ou d'une broche, le poussoir (3) étant dimensionné de telle sorte qu'il peut être inséré dans le tube applicateur (1), et
dans lequel le tube applicateur (1) présente une pointe biseautée (11), une partie principale cylindrique creuse (12) et une pièce d'extrémité en forme de bride (13).

2. Ensemble selon la revendication précédente,
dans lequel la pointe biseautée (11) présente au moins deux extrémités de pointe (21, 22) qui sont formées par des évidements (17, 18) dans le tube applicateur (1) ; et/ou
dans lequel la pointe (11) du tube applicateur (1) est biseautée de manière asymétrique en rotation par rapport à l'axe longitudinal (X) du tube applicateur (1).

3. Ensemble selon l'une quelconque des revendications 1 à 2,
dans lequel la pointe biseautée (11) est biseautée de manière asymétrique en rotation par rapport à l'axe longitudinal (X) du tube applicateur (1) et présente deux extrémités de pointe (21, 22) ; et
dans lequel les deux extrémités de pointe (21, 22) et au moins une parallèle à l'axe longitudinal (X) du tube applicateur (1) passant à travers celles-ci définissent au moins un plan de pointe (E1, E2) ;
dans lequel, par rapport à un plan de pointe (E1), un biseautage supérieur (14) de la pointe (11) est décrit géométriquement par une intersection avec un plan supérieur (14) incliné d'un angle aigu supérieur α par rapport à ce plan de pointe (E1) ; et/ou
dans lequel, par rapport au même plan de pointe ou à un autre plan de pointe (E2), un biseautage inférieur (15) de la pointe (11) est décrit géométriquement par une intersection avec un plan inférieur (15) incliné d'un angle aigu inférieur β par rapport à ce plan de pointe (E2) .

4. Ensemble selon la revendication précédente,
dans lequel l'angle aigu supérieur α est tel que :
20° ≤ α ≤ 30°, notamment α = 25° ; et/ou
dans lequel l'angle aigu inférieur β est tel que :
20° ≤ β ≤ 30°, notamment β = 25°.

5. Ensemble selon l'une quelconque des revendications 2 à 4, dans lequel les deux extrémités de pointe (21, 22) présentent, dans une direction orthogonale à l'axe longitudinal (X) du tube applicateur (1), une hauteur h telle que :
0,5 mm ≤ h ≤ 1,5 mm, notamment h = 1,0 mm.

6. Ensemble selon l'une quelconque des revendications précédentes, dans lequel le tube guide-fil de forage (2) présente une partie principale cylindrique creuse (201) et une pièce d'extrémité en forme de bride (202).

7. Ensemble selon l'une quelconque des revendications précédentes,
dans lequel le poussoir (3) présente un mandrin (301) à son extrémité proximale, une partie principale cylindrique (302) et une partie de tête de poussoir (303) à son extrémité distale ; et/ou
dans lequel une longueur LSK de la partie de tête de poussoir (303) le long de l'axe longitudinal (Z) du poussoir (3) est telle que : 13 mm ≤ LSK ≤ 17 mm, notamment LSK = 15mm ; et/ou
dans lequel un diamètre extérieur DSK de la partie de tête de poussoir (303) est tel que :
8 mm ≤ DSK ≤ 12 mm, notamment DSK = 10mm ; et/ou
dans lequel la longueur LD du mandrin (301) le long de l'axe longitudinal (Z) du poussoir (3) est telle que :
1,9 mm ≤ LD ≤ 2, 1 mm, notamment LD = 2,0mm ; et/ou
dans lequel le diamètre extérieur DD du mandrin (301) est tel que :
0,9 mm ≤ DD ≤ 1,1 mm, notamment DD = 1,0 mm.

8. Ensemble selon l'une quelconque des revendications précédentes,
dans lequel un diamètre extérieur DA du tube applicateur (1) est tel que :
3,9 mm ≤ DA ≤ 4, 1 mm, notamment DA = 4,0 mm ; et
dans lequel un diamètre intérieur dA du tube applicateur (1) est tel que :
3,3 mm ≤ dA ≤ 3,5 mm, notamment dA = 3,4 mm ; et
dans lequel un diamètre extérieur DB du tube guide-fil de forage (2) est tel que :
2,9 mm ≤ DB ≤ 3, 1 mm, notamment DB = 3,0 mm ; et
dans lequel un diamètre intérieur dB du tube guide-fil de forage (2) est tel que :
2,3 mm ≤ dB ≤ 2,5 mm, notamment dB = 2,4 mm ; et
dans lequel un diamètre extérieur DS du poussoir (3) est tel que :
2,9 mm ≤ DS ≤ 3, 1 mm, notamment DS = 3,0 mm.

9. Ensemble selon l'une quelconque des revendications précédentes,
dans lequel une longueur LA du tube applicateur (1) est telle que :
250 mm ≤ LA ≤ 290 mm, notamment LA = 270 mm ; et
dans lequel une longueur LB du tube guide-fil de forage (2) est telle que :
245 mm ≤ LB ≤ 285 mm, notamment LB = 265 mm ; et
dans lequel une longueur LS du poussoir (3) est telle que :
265 mm ≤ LS ≤ 305 mm, notamment LS = 285 mm ; et
dans lequel en outre : LB < LA < LS ; et
dans lequel les longueurs LA, LB et LS indiquées sont déterminées, en présence d'une pièce d'extrémité en forme de bride (13, 202) ou d'une partie de tête (303) sur le composant respectif (1, 2, 3), sans la pièce d'extrémité en forme de bride respective (13, 202) ou sans la partie de tête (303).

10. Ensemble selon l'une quelconque des revendications précédentes,
dans lequel une longueur LFA d'une pièce d'extrémité en forme de bride (13) du tube applicateur (1) le long de l'axe principal (X) du tube applicateur (1) et un diamètre extérieur DFA de la pièce d'extrémité en forme de bride (13) du tube applicateur (1) sont tels que : 2,9 mm ≤ LFA ≤ 3, 1 mm, notamment LFA = 3 mm, ainsi que 9 mm ≤ DFA ≤ 11 mm, notamment DFA = 10mm ; et/ou
dans lequel une longueur LFB d'une pièce d'extrémité en forme de bride (202) du tube guide-fil de forage (2) le long de l'axe principal (Y) du tube guide-fil de forage (2) et un diamètre extérieur DFB de la pièce d'extrémité en forme de bride (202) du tube guide-fil de forage (2) sont tels que : LFB = LFA ± 0,1 mm, notamment LFB = 3 mm, ainsi que DFB ≥ DFA, notamment DFB = 12 mm.

11. Ensemble selon l'une quelconque des revendications précédentes, dans lequel les constituants de l'ensemble sont réutilisables après une stérilisation appropriée.

12. Ensemble selon l'une quelconque des revendications précédentes, qui présente en outre l'élément suivant :
un fil de forage, notamment un fil en K, pour préforer un trou à travers un implant dans la partie osseuse d'un corps vertébral.

13. Ensemble selon l'une quelconque des revendications précédentes, qui présente en outre l'élément suivant :
un implant à insérer dans un disque intervertébral.

14. Ensemble selon la revendication précédente, dans lequel l'implant est poreux, spongieux, compact, en feutre, non tissé ou réticulé.

15. Ensemble selon l'une quelconque des revendications 13 à 14, dans lequel l'implant présente au moins l'un des matériaux suivants :
- du collagène ;
- du hyaluronane (acide hyaluronique) ;
- de l'acide polyglycolique, de l'acide polylactique et/ou leurs copolymères respectifs ;
- de la polycaprolactone ;
- une membrane naturelle, notamment du périoste, du périchondre et/ou des fascias, ainsi que leurs modifications ;
- un alginate ;
- de l'agarose ;
- de la fibrine ;
- un matériau contenant de l'albumine ;
- des polysaccharides,
ainsi que leurs combinaisons.

16. Ensemble selon l'une quelconque des revendications précédentes, qui présente en outre l'élément suivant :
au moins un clou ou une broche, de préférence résorbable, pour la fixation d'un implant dans un disque intervertébral.

17. Ensemble selon la revendication précédente,
dans lequel le clou ou la broche, de préférence résorbable, présente un diamètre DN tel que : 1,4 mm ≤ DN ≤ 1,6 mm, notamment DN = 1,5 mm ; et/ou
dans lequel le clou ou la broche résorbable présente une longueur LN telle que : 10 mm ≤ LN ≤ 30 mm.

18. Ensemble selon l'une quelconque des revendications précédentes,
dans lequel le tube applicateur (1) peut être enfiché sur un endoscope ; et/ou
dans lequel aucune autre fixation, notamment aucun assemblage par vis, n'est nécessaire pour la liaison entre un endoscope et le tube applicateur (1) ; et/ou
dans lequel le tube applicateur (1) ne présente pas de support ou de poignée séparé.
